# EUROPÄISCHE PATENTSCHRIFT

(11) **EP 0 884 987 B1**
(45) Veröffentlichungstag und Bekanntmachung des Hinweises auf die Patenterteilung: **25.06.2003**
(21) Anmeldenummer: 97949871.4
(22) Anmeldetag: 28.10.1997
(51) Int. Cl.: A61F 2/06

(54) **EXPANDIERBARE INTRALUMINALE VORRICHTUNG**
EXPANDABLE INTRALUMINAL DEVICE
DISPOSITIF INTRALUMINAL EXTENSIBLE

(30) Priorität: 28.10.1996 DE 19645292; 10.12.1996 DE 19653717
(43) Veröffentlichungstag der Anmeldung: 23.12.1998
(73) Patentinhaber: BIOTRONIK Mess- und Therapiegeräte GmbH & Co Ingenieurbüro Berlin, 12359 Berlin (DE)
(72) Erfinder: SCHMITZ, Klaus-Peter, D-18119 Rostock (DE); BEHREND, Detlef, D-18119 Rostock (DE)
(74) Vertreter: Christiansen, Henning, Dipl.-Ing.
(86) Internationale Anmeldenummer: DE9702570
(87) Internationale Veröffentlichungsnummer: WO98018405

(56) Entgegenhaltungen:
- DE-A- 4 303 181
- DE-C- 4 429 380
- US-A- 5 695 516

## Beschreibung

Die Erfindung betrifft eine expandierbare intraluminale Vorrichtung entsprechend der im Oberbegriff des ersten Anspruchs genannten Art. Eine solche Vorrichtung ist aus DE-A-4 303 181 bekannt.

Aus den europäischen Patentschriften EU-B1 0 364 787 und EU-B1 335 341 ist ein aufweitbares intraluminales Gewebe mit mindestens einem dünnwandigen, rohrförmigen Teil (nachfolgend als Stent bezeichnet) bekannt. Die Mantelfläche des Stents ist netzförmig ausgebildet und weist in axialer Richtung in Reihe und in Umfangsrichtung gegeneinander versetzt angeordnete, schlitzförmige eine Ausnehmung umschließende expandierbare Steganordnungen auf, welche durch sich geradlinig in axialer und Umfangsrichtung erstreckende Stege von geringer Materialstärke begrenzt sind. Die Stege bilden an den Verbindungsstellen jeweils die Schenkel eines rechten Winkels.

Bei Einwirkung einer von innen nach außen gerichteten Kraft, beispielsweise durch einen mit Druckgas beaufschlagten, schlauchförmigen Dilator, expandiert der Stent unter grundsätzlicher Beibehaltung seiner Rohrform. Der expandierte Stent ist im Vergleich zu dem nichtexpandierten Stent jedoch erheblich kürzer und seine netzförmig strukturierte Mantelfläche weist im wesentlichen wabenförmig ausgebildete eine Ausnehmung umschließende expandierbare Steganordnungen auf, welche durch Verformen der sich zuvor in axialer Richtung erstreckenden Stege entstehen.

Der vorstehend beschriebene rohrförmige, aus aufweitbarem Gewebe bestehende und zum Aufweiten eines Hohlorgans verwendbare Stent weist jedoch den Nachteil auf, daß beim Expandieren aufgrund der Verformung der sich axial erstrekkenden Stege eine erhebliche Verkürzung in axialer Richtung eintritt.

Ausgehend von den Mängeln des Standes der Technik liegt der Erfindung die Aufgabe zugrunde, eine expandierbare intraluminale Vorrichtung der eingangs genannten Gattung (Stent) anzugeben, welche sich beim Expandieren, bezogen auf einen bestimmten Wert der Durchmesservergrößerung, in axialer Richtung nicht - oder nur unwesentlich verkürzt.

Die Aufgabe wird durch die kennzeichnenden Merkmale des Anspruchs 1 gelöst.

Die Erfindung schließt die Erkenntnis ein, daß bei einem hohlzylindrischen, durch Expansion im Durchmesser vergrößerbarer Körper mit einer verformbaren und mit schlitzartigen, reihenförmig angeordnete, durch sich im wesentlichen in Umfangsrichtung bzw. axial erstreckende Stege begrenzten eine Ausnehmung umschließenden expandierbaren Steganordnungen gebildeten Mantelfläche, beim Expandieren um ein bestimmtes Maß dann im wesentlichen keine axiale Verkürzung eintritt, wenn die Steganordnungen eine durch eine Einschnürung gebildete Expansionsreserve aufweisen. Eine derartige Einschnürung ist erreichbar, wenn die sich in axialer Richtung erstreckenden Stege eine Knickung entgegen der Dehnungsrichtung bei einer Expansion des hohlzylindrischen Körpers aufweisen. Dadurch vergrößert sich in günstiger Weise der Schwenkwinkel und die sich axial erstreckenden, in zwei Abschnitte untergliederten Stege übernehmen den wesentlichen Anteil der Verformung der Mantelfläche, wenn der Stent expandiert wird.

Die geknickten, sich axial erstreckenden Stege, bei denen entsprechend einer bevorzugten Ausführungsform der Erfindung die Enden der sich in Umfangsrichtung erstreckenden Stege im Knickpunkt befestigt sind, bilden somit eine Expansionsreserver in Umfangsrichtung. Diese kann für eine Durchmessersvergrößerung des hohlzylindrischen Körpers genutzt werden, ohne daß bei diesem eine Verkürzung in axialer Richtung eintritt.

Entsprechend einer bevorzugten Ausführungsform der Erfindung ist die expandierbare intraluminale Vorrichtung zum Aufweiten eines Hohlorgans, beispielsweise eines Blutgefäßes, als dünnwandiger Hohlzylinder mit einer netzförmigen Mantelfläche ausgebildet, deren Abwicklung in der Ebene bei nicht expandierter Vorrichtung in axialer Richtung reihenförmig angeordneten eine Ausnehmung umschließenden expandierbaren Steganordnungen in der Form zweier gleichschenkliger, spiegelsymmetrisch einander gegenüberliegend angeordneter Trapeze aufweist, wobei die kurze Grundlinie der Trapeze die Symmetrieachse bildet.

Die Durchmesservergrößerung des hohlzylindrischen Stents ohne Verringerung seiner axialen Länge ist begrenzt. Der Stentdurchmesser kann bei dieser Längenoption maximal verdoppelt werden, wenn die Länge der kurzen Grundlinie der Trapeze bei maximaler Einschnürung der sich axial erstrekkenden eine Ausnehmung umschließende expandierbare Steganordnungen gegen Null tendiert.

Günstige Expansionsergebnisse, welche im wesentlichen zu einer Verdopplung des Stentdurchmessers führen, sind mit einer Vorrichtung mit einer in vorzugsweise vier sich in axialer Richtung erstreckenden Reihen von eine Ausnehmung umschließende expandierbare Steganordnungen erreichbar, bei denen die kleine Grundseite der Trapeze und die sich in Umfangsrichtung erstreckenden Koppelglieder ein Längenverhältnis von vorzugsweise eins zu acht aufweisen. Für den Stent ist im unexpandierten Zustand eine Stentlänge in einem Bereich von 4,5 bis 6,5 mm, vorzugsweise von 5,78 mm, und ein Stentdurchmesser im Bereich von 1,2 mm bis 1,8 mm, vorzugsweise von 1,5 mm vorgesehen.

Nach einer vorteilhaften Weiterbildung der bevorzugten Ausführungsform der Erfindung weisen die sich in Umfangsrichtung erstreckenden Stege der expandierbaren Vorrichtung eine größere Materialstärke auf als die sich in axialer Richtung erstreckenden Stege, wodurch in günstiger Weise erreicht wird, daß sich bei der durch Expansion ausgelösten Expansion der Vorrichtung zuerst die sich in axialer Richtung erstreckenden Stege verformen, ohne daß eine nennenswerte Streckung der sich in Umfangsrichtung erstreckenden Stege zu verzeichnene ist. Dabei bilden die eine Ausnehmung umschließenden expandierbaren Steganordnungen bei zwischenzeitlichem axialem Längenzuwachs des Stents eine im wesentlichen rechteckige Form. Danach werden die Steganordnungen bei weiterer Krafteinwirkung in eine Wabenform überführt, wobei der Stent in vorteilhafter Weise bei vergrößertem Durchmesser erneut seine ürsprüngliche Länge einnimmt.

Entsprechend einer anderen vorteilhaften Ausführungsform der Erfindung ist zur Erzeugung der Einschnürung der eine Ausnehmung umschließende expandierbare Steganordnungen eine Mehrfach-Knickung der sich axial erstreckenden Stege vorgesehen, welche dadurch eine Untergliederung in fünf symmetrisch angeordnete Abschnitte aufweisen. Dadurch ist auf einfache Weise eine Reduzierung der für die Expansion aufzubringenden Kräfte erreichbar, da sich in Abschnitte unterteilten Stege leichter verformen lassen.

Um bei der expansionsbedingten Verformung der Mantelfläche des erfindungsgemäßen Stents das Auftreten von lokalen Extremwerten der Biegespannung in dem verwendeten Material zu vermeiden und somit einen möglichen Materialbruch vorzubeugen, sind an den Verbindungsstellen zwischen den einzelnen Stegen Materialverrundungen vorgesehen.

Der erfindungsgemäße Stent besteht nach einer vorteilhaften Weiterbildung der Erfindung aus Titan, Tantal oder einem anderen biokompatibelen Werkstoff bzw. einer entsprechenden Legierung.

Entsprechend einer anderen günstigen Weiterbildung der Erfindung weist der erfindungsgemäße Stent eine biokompatible Beschichtung aus Siliziumcarbid auf, welche einer Throbogenität des Implantats entgegenwirkt.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Darstellung einer bevorzugten Ausführungsform der Erfindung in Form einer Abwicklung der Mantelfläche im unexpandierten Zustand, wobei die Achse des Stents für den Betrachter senkrecht gerichtet ist,
- Figur 2: die Abwicklung der Mantelfläche der Vorrichtung gemäß Figur 1 nach erfolgter Expansion, wobei die Achse des Stents für den Betrachter ebenfalls senkrecht gerichtet ist, sowie
- Figur 3: eine andere vorteilhafte Ausführungsform der Erfindung, wobei die Achse des Stents für den Betrachter waagerecht gerichtet ist.

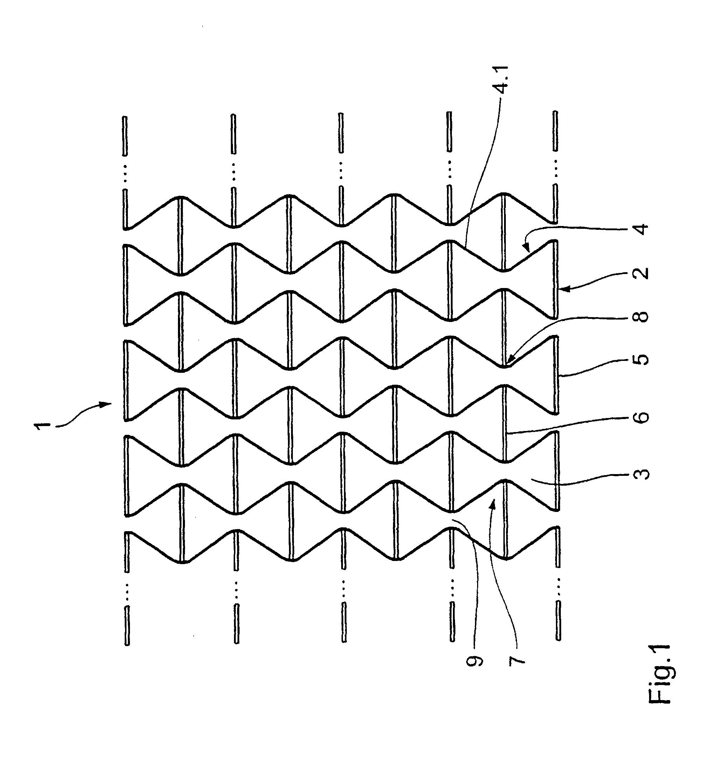

Die in Figur 1 dargestellte Konstruktion zeigt die Abwicklung 1 der Mantelfläche eines erfindungsgemäßen (rohrförmigen) Stents in unexpandiertem Zustand. Die Abwicklung weist in Reihen 2 angeordnete eine Ausnehmung umschließende expandierbare Steganordnungen 3 auf, welche durch Koppelglieder 6 miteinander verbunden sind. Es sind vier gleichartig ausgebildete Reihen 2 vorgesehen, wobei am oberen und unteren Ende der Abwicklung 1 aus Gründen der bessereren zeichnerischen Darstellung jeweils eine halbe Reihe angeordnet ist.

Jede der reihenförmig angeordneten eine Ausnehmung umschließenden expandierbaren Steganordnungen 3 weist eine Einschnürung 7, welche durch Knicken der sich im wesentlichen axial erstreckenden Stege 4 gebildet wird. Das Knicken teilt die Stege 4 in zwei gleichlange Abschnitte 4.1, welche die Seiten eines Trapezes bilden. Die Fläche der eine Ausnehmung umschließende expandierbare Steganordnungen 3 weist damit die Form von zwei gleichschenkligen, spiegelsymmetrisch einander gegenüberliegend angeordneten Trapezen mit den Grundlinien 5 und 9 auf, wobei die gemeinsame kurze Grundlinie 9 der Trapeze die Symmetrieachse bildet. Die Steganordnung hat also die Form einer "Eieruhr".

Die einander in Umfangsrichtung gegenüberliegenden eine Ausnehmung umschließende expandierbare Steganordnungen 3 der benachbart angeordneten Reihen 2 sind durch die Koppelglieder 6 miteinander verbunden, wobei der Befestigungspunkt 8 der Koppelglieder 6 mit dem Knickpunkt der Stege 4 der Steganordnungen 3 übereinstimmt. Die Koppelglieder 6 weisen eine größere Materialstärke auf als die geknickten Stege 4, so daß die Verformung der Mantelfläche beim Expandieren des Stents im wesentlichen vollständig über die Stegen 4 erfolgt.

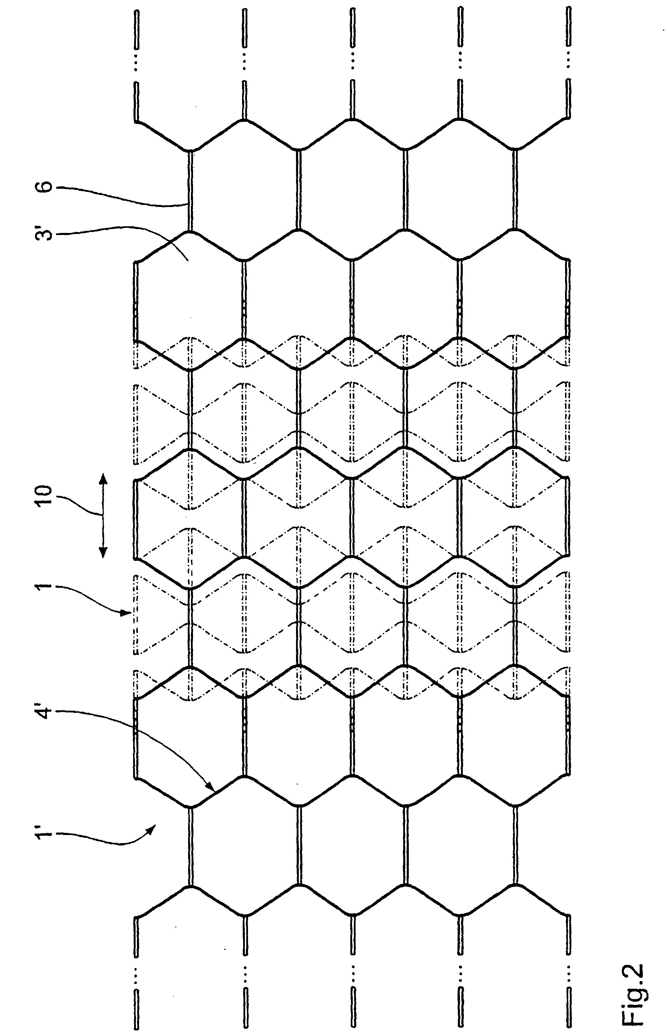

In Figur 2 ist die Abwicklung 1' der Mantelfläche des Stents nach erfolgter Expansion dargestellt, wobei die Dehnungsrichtung mit 10 gekennzeichnet und zum Vergleich die Abwicklung der Mantelfläche 1 gemäß Figur 1 in Strichliniendarstellung gezeigt ist.

Die Koppelglieder 6 zeigen keine Veränderungen, wogegen die wabenförmigen Steganordnungen 3' der Mantelfläche des expandierten Stents unter zwischenzeitlicher Annahme einer Rechteckform aus den Steganordnungen (vergleiche die Position 3 in Figur 1) hervorgegangen sind. Die Verrundungen an den Verbindungspunkten zwischen den Stegen 4' und den Koppelgliedern 6 erleichtern die Dehnung und beugen der Bruchgefahr durch lokale Extremwerte der Spannung beim Expandieren des Stents vor.

Durch das Aufheben der Einschnürung der eine Ausnehmung umschließende expandierbare Steganordnungen (vergleiche die Positionen 3 und 7 in Figur 1) verfügbare Expansionsreserve ermöglicht eine Durchmesservergrößerung des Stents auf nahezu das Doppelte des Ausgangsdurchmessers, ohne das sich das Längenmaß des Stent verringert. Die Form der "Eieruhr" wird also jeweils zu der eines Fasses aufgeweitet. Da die tangential gerichteten Koppelelemente im Bereich der Taille der Eieruhr angreifen, kommt die mit der Ausdehnung zu einem Faß verbundene Ausdehnung der Expansion des Stents zugute.

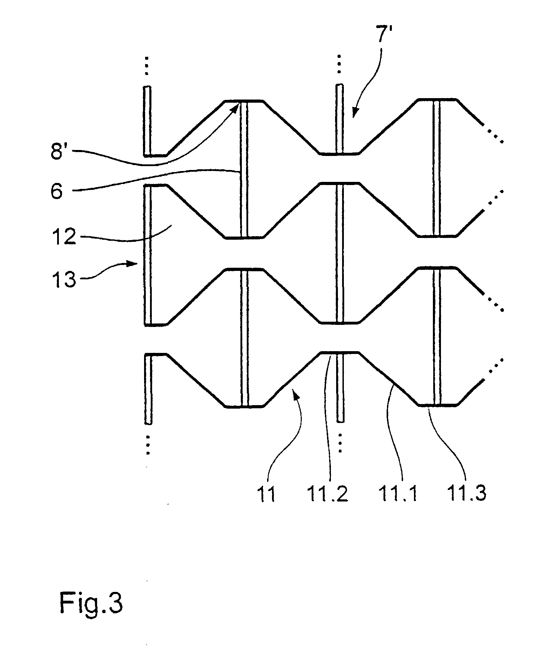

Der in Figur 3 dargestellte Teilbereich der Abwicklung einer Mantelfläche eines unexpandierten Stents weist eine Ausnehmung umschließende expandierbare Steganordnungen 12 mit einer Einschnürung 7' auf, welche durch eine Mehrfach-Knickung der sich in axialer Richtung erstreckenden Stege 11 bedingt ist. Die Stege 11 sind in Abschnitte 11.1, 11.2 und 11.3 unterteilt, wobei jeweils zwei Abschnitte 11.1, 11.3 aus Symmetriegründen die gleiche Länge aufweisen. Die sich in Umfangsrichtung erstreckenden Koppelstege 6 sind in der Mitte des sich axial erstreckenden Abschnitts 11.2 der Stege 11 am Befestigungspunkt 8' mit den jeweils nebeneinander angeordneten jeweils eine Ausnehmung umschließende expandierbare Steganordnungen 12 der in Umfangsrichtung benachbarten Reihen 13 verbunden.

Durch die Mehrfach-Knickung der Stege 11 ist die beim Expandieren des Stents in den Ausführungen zu Figur 2 beschriebene Durchmesservergrößerung mit geringerem Kraftaufwand erreichbar und die Gefahr des Auftretens lokaler extremer mechanisch Spannungen wird verhindert.

Die Erfindung beschränkt sich in ihrer Ausführung nicht auf die vorstehend angegebenen bevorzugten Ausführungsbeispiele. Vielmehr ist eine Anzahl von Varianten günstig, welche von der dargestellten Lösung auch bei grundsätzlich anders gearteten Ausführungen Gebrauch macht.

## Patentansprüche

1. Expandierbare intraluminale Vorrichtung (1) zum Aufweiten eines Gefäßes, welche im wesentlichen die Form eines dünnwandigen Hohlzylinders aufweist, dessen Mantelfläche netzförmig ausgebildet ist und durch Stege (4, 5) begrenzte, jeweils eine Ausnehmung umschließende expandierbare Steganordnungen (3) aufweist, wobei die sich axial erstrekkenden Reihen (2) von Steganordnungen durch sich in Umfangsrichtung erstreckende stegförmige Koppelglieder (6) miteinander verbunden und die Koppelglieder (6) jeweils in der Mitte der sich axial erstreckenden Stege (4) von in Umfangsrichtung benachbart angeordneten Steganordnungen (3) befestigt sind,
**dadurch gekennzeichnet,**
**daß** die in axialen Reihen (2, 13) angeordneten, jeweils eine Ausnehmung umschließenden expandierbaren Steganordnungen (3, 12) eine im wesentlichen in Umfangsrichtung gerichtete Einschnürung (7, 7') bei nicht expandiertem Zustand aufweisen.

2. Vorrichtung nach Anspruch 1, **gekennzeichnet durch** eine **durch** Knickung der sich in axialer Richtung erstreckenden Stege (4, 11) erzeugte Einschnürung (7, 7').

3. Vorrichtung nach Anspruch 2, **dadurch gekennzeichnet, daß** eine Mehrfach-Knickung vorgesehen ist.

4. Vorrichtung nach Anspruch 2, **dadurch gekennzeichnet, daß** der Knickpunkt (8) der Stege (4) mit dem Befestigungspunkt der Koppelglieder (6) übereinstimmt.

5. Vorrichtung nach Anspruch 1, **dadurch gekennzeichnet, daß** die in Reihen (2) angeordneten jeweils eine Ausnehmung umschließende expandierbare Steganordnungen (3) einer unexpandierten Abwicklung der Mantelfläche die Form von zwei gleichschenkligen, spiegelsymmetrisch einander gegenüberliegend angeordneten Trapezen aufweisen, wobei die gemeinsame kurze Grundlinie (9) der Trapeze die Symmetrieachse bildet.

6. Vorrichtung nach Anspruch 5, **dadurch gekennzeichnet, daß** die Länge der kurze Grundlinie (9) wesentlich geringeren ist als die Länge der stegförmigen Koppelglieder (6).

7. Vorrichtung nach Anspruch 6, **dadurch gekennzeichnet**, die Länge der Koppelglieder (6) der achtfachen Länge der kurzen Grundlinie (9) entspricht.

8. Vorrichtung nach einem der vorhergehende Ansprüche, **dadurch gekennzeichnet, daß** die Koppelglieder (6) und die sich in Umfangsrichtung erstreckenden Stege (5) eine größere Materialstärke aufweisen, als die sich in axialer Richtung erstreckenden, geknickten Stege (4, 11).

9. Vorrichtung nach Anspruch 8, **gekennzeichnet durch** eine bezogen auf die sich in axialer Richtung erstreckenden Stege (4, 11) doppelte Materialstärke der Koppelglieder (6) bzw. der sich in Umfangsrichtung erstreckenden Stege (5).

10. Vorrichtung nach Anspruch 1, **gekennzeichnet durch** eine Länge in einem Bereich von 4,5 bis 6,5 mm, vorzugsweise von 5,78 mm, und einen Durchmesser in einem Bereich von 1,2 mm bis 1,8 mm, vorzugsweise von 1,5 mm.

11. Vorrichtung nach Anspruch 1, **dadurch gekennzeichnet, daß** die jeweils eine Ausnehmung umschließenden expandierbaren Steganordnungen (3, 12) an ihren Eckpunkten und an dem Befestigungspunkten der Koppelglieder (6) verrundet ausgebildet sind.

12. Vorrichtung nach Anspruch 1, **gekennzeichnet durch** eine Herstellung aus Titan, Tantal oder einem anderen biokompatiblen Metall bzw. einer entsprechenden Legierung.

13. Vorrichtung nach Anspruch 12, **gekennzeichnet durch** eine biokompatible Beschichtung.

14. Vorrichtung nach Anspruch 13, **dadurch gekennzeichnet, daß** eine Beschichtung aus amorphem Siliziumcarbid vorgesehen ist.

## Claims

1. An expandable intraluminal device (1) for widening a vessel, which substantially has the shape of a thin-walled hollow cylinder, the surface area of which has a net-shaped design and comprises expandable web configurations (3) that are limited by webs (4, 5) and each enclose a recess, the axially extending rows (2) of web configurations being connected to each other by web-shaped coupling members (6) extending in the peripheral direction and the coupling members (6) each being attached in the centre of the axially extending webs (4) of web configurations (3) disposed adjacently in the peripheral direction,
**characterised in that** in the non-expanded state the expandable web configurations (3, 12) that are disposed in axial rows (2, 13) and each enclose a recess have a constriction (7, 7') directed substantially in the peripheral direction.

2. A device according to Claim 1,
**characterised by** a constriction (7, 7') produced by bending the webs (4, 11) that extend in the axial direction.

3. A device according to Claim 2,
**characterised in that** repeated bending is provided.

4. A device according to Claim 2,
**characterised in that** the bending point (8) of the webs (4) coincides with the attachment point of the coupling members (6).

5. A device according to Claim 1,
**characterised in that** the expandable web configurations (3), which are disposed in rows (2) and each enclose a recess, of an unexpanded developed view of the surface area have the shape of two equilateral trapeziums which are disposed opposite each other with mirror symmetry, with the common short base (9) of the trapezium forming the axis of symmetry.

6. A device according to Claim 5,
**characterised in that** the length of the short base (9) is substantially less than the length of the web-shaped coupling members (6).

7. A device according to Claim 6,
**characterised in that** the length of the coupling members (6) corresponds to eight times the length of the short base (9).

8. A device according to one of the precharacterising Claims,
**characterised in that** the coupling members (6) and the webs (5) extending in the peripheral direction have a greater material thickness than the bent webs (4, 11) extending in the axial direction.

9. A device according to Claim 4,
**characterised by** a material thickness of the coupling members (6) and of the webs (5) extending in the peripheral direction which is double that of the webs (4, 11) extending in the axial direction.

10. A device according to Claim 1,
**characterised by** a length in a range from 4.5 to 6.5 mm, preferably of 5.78 mm, and a diameter in a range from 1.2 to 1.8 mm, preferably of 1.5 mm.

11. A device according to Claim 1,
**characterised in that** the expandable web configurations (3, 12), each of which enclose a recess, are rounded at their corner points and at the attachment points of the coupling members (6).

12. A device according to Claim 1,
**characterised by** production from titanium, tantalum or another biocompatible metal or a corresponding alloy.

13. A device according to Claim 12,
**characterised by** a biocompatible coating.

14. A device according to Claim 13,
**characterised in that** a coating of amorphous silicon carbide is provided.

## Revendications

1. Dispositif intraluminal expansible (1) pour évaser un vaisseau, qui présente sensiblement la forme d'un cylindre creux à paroi mince dont la surface enveloppe est réalisée en forme de treillis et comprend des agencements de barrettes expansibles (3) limités par des barrettes (4, 5) et entourant chacun un évidement, les rangées (2) s'étendant axialement d'agencements de barrettes étant reliées les unes aux autres par des organes d'accouplement (6) en forme de barrette s'étendant en direction périphérique et les organes d'accouplement (6) étant fixés chacun au milieu des barrettes axiales (4) d'agencements de barrettes (3) disposés au voisinage en direction périphérique,
**caractérisé en ce que**
les agencements de barrettes expansibles (3, 12) agencés en rangées axiales (2, 13) et entourant chacun un évidement présentent, dans l'état non expansé, un rétrécissement (7, 7') dirigé sensiblement en direction périphérique.

2. Dispositif selon la revendication 1, **caractérisé par** un rétrécissement (7, 7') généré par un pliage des barrettes (4, 11) s'étendant en direction axiale.

3. Dispositif selon la revendication 2, **caractérisé en ce qu'**il est prévu un pliage multiple.

4. Dispositif selon la revendication 2, **caractérisé en ce que** le point de pliage (8) des barrettes (4) coïncide avec le point de fixation des organes d'accouplement (6).

5. Dispositif selon la revendication 1, **caractérisé en ce que** les agencements de barrettes expansibles (3), disposés en rangées (2) et enfermant chacun un évidement, d'un développement non expansé de la surface enveloppe présentent la forme de deux trapèzes isocèles agencés symétriquement à l'opposé l'un de l'autre, la petite ligne de base commune (9) des trapèzes formant l'axe de symétrie.

6. Dispositif selon la revendication 5, **caractérisé en ce que** la longueur de la petite ligne de base (9) est considérablement inférieure à la longueur des organes d'accouplement (6) en forme de barrette.

7. Dispositif selon la revendication 6, **caractérisé en ce que** la longueur des organes d'accouplement (6) est huit fois la longueur de la petite ligne de base (9).

8. Dispositif selon l'une des revendications précédentes, **caractérisé en ce que** les organes d'accouplement (6) et les barrettes (5) s'étendant en direction périphérique présentent une épaisseur de matière supérieure à celle des barrettes pliées (4, 11) s'étendant en direction axiale.

9. Dispositif selon la revendication 8, **caractérisé par** une épaisseur de matière des organes d'accouplement (6) ou des barrettes (5) s'étendant en direction périphérique, qui est deux fois plus élevée que celle des barrettes (4, 11) s'étendant en direction axiale.

10. Dispositif selon la revendication 1, **caractérisé par** une longueur dans la plage de 4,5 à 6,5 mm, de préférence de 5,78 mm, et par un diamètre dans une plage de 1,2 à 1,8 mm, de préférence de 1,5 mm.

11. Dispositif selon la revendication 1, **caractérisé en ce que** les agencements de barrette expansibles (3, 12) entourant chacun un évidement sont réalisés arrondis à leurs coins et aux points de fixation des organes d'accouplement (6).

12. Dispositif selon la revendication 1, **caractérisé par** une fabrication en titane, tantale ou en un autre métal biocompatible ou en un alliage correspondant.

13. Dispositif selon la revendication 12, **caractérisé par** un revêtement biocompatible.

14. Dispositif selon la revendication 13, **caractérisé en ce qu'**il est prévu un revêtement en carbure de silicium amorphe.
